# EUROPEAN PATENT SPECIFICATION

(11) **EP 2 334 264 B1**
(45) Date of publication and mention of the grant of the patent: **04.11.2020**
(21) Application number: 09783011.1
(22) Date of filing: 15.09.2009
(51) Int. Cl.: A61F 2/966

(54) **STENT DEVICE DELIVERY SYSTEM**
STENTABLAGESYSTEM
SYSTÈME DE POSE DE DISPOSITIF DE STENT

(30) Priority: 16.09.2008 GB 0816965
(43) Date of publication of application: 22.06.2011
(73) Proprietor: Angiomed GmbH & Co. Medizintechnik KG, 76227 Karlsruhe (DE)
(72) Inventor: DORN, Jürgen, 68809 Neulußheim (DE); HOFFMANN, Martina, 76297 Stutensee (DE); WALTER, Beate, 76297 Stutensee (DE)
(74) Representative: Hoffmann Eitle
(86) International application number: PCT/EP2009/061918
(87) International publication number: WO 2010/031755

(56) References cited:
- EP-A- 1 382 367
- EP-A- 1 466 570
- EP-A1- 1 382 367
- EP-A1- 1 466 570
- EP-A1- 1 690 512
- WO-A-00/71058
- WO-A-2006/026377
- WO-A-2007/149464
- WO-A1-00/71058
- WO-A1-2004/096091
- WO-A1-2006/026377
- WO-A2-2007/149464
- US-A- 6 048 350
- US-A- 6 048 350
- US-A- 6 110 180
- US-A- 6 110 180
- US-A1- 2001 049 549
- US-A1- 2001 049 549
- US-A1- 2003 032 999
- US-A1- 2003 032 999
- US-A1- 2004 143 272
- US-A1- 2004 143 272

## Description

### Field of the Invention

This invention relates in one aspect to a method of loading a self-expanding stent device into a delivery sheath, in which the stent device, in a radially contracted delivery configuration, is axially held on an inner catheter and advanced axially into the sheath for delivery to a stenting site in which the sheath is withdrawn to release the stent device for radial expansion. In another aspect, the invention relates to a stent device delivery system comprising a self-expanding stent device within a percutaneous transluminal delivery sheath, where the sheath withdraws proximally to release the stent device at a stenting site, while an inner catheter within the stent device retains the stent device at the site during withdrawal of the sheath.

### Background of the invention

A stent device is a tubular vascular implant that has structure able to support a segment of a blood vessel or other anatomical lumen against collapse, while allowing blood or other bodily fluid to flow through the lumen of the stent device. The stent device is delivered with a delivery catheter to the site where a diseased segment of blood vessel is located and deployed there to support the blood vessel against radial collapse. The stent device is advanced to the site in a collapsed configuration and expanded to contact the inner wall of the blood vessel upon deployment.

There are stent devices that require forced expansion such as by inflating a balloon inside the lumen of the stent device and self-expanding states that are so made that they automatically expand to the radially expanded configuration once given the radial freedom to do so; that is once the stent device is unconstrained. It is with this latter type of stent device that the present disclosure is concerned.

A stent device includes a tubular framework that is resistant to radial compression so that the blood vessel is maintained open. The stent device may include a cover on the inner and/or the outer surface of the framework, in which case the stent device is often termed a stent graft. If the framework is without inner and outer coverings, the stent device may be labelled a bare stent.

One suitable material for making the framework of the stent device is the nickel titanium shape memory alloy known as NITINOL. Such stents may be put into a collapsed configuration at a low temperature and a memory of a radially expanded configuration is maintained. The nickel titanium material is biologically compatible and returns to the expanded configuration between room temperature and body temperature.

A self-expanding stent device is subjected to axial forces during loading of the stent device into an outer sheath of a delivery system and also during deployment of the stent device from the outer sheath of the delivery system to a site of a vascular lumen where it is to be implanted. During these procedures, the stent device is held axially in position by a stent device pusher and the outer sheath is moved axially relative to the stent device and stent device pusher. The stent device pusher may be the same element for the loading procedure as well as the deployment procedure. The stent device is loaded into the outer sheath in a crimped state. Accordingly, the outer sheath constrains radial expansion of the stent device. Therefore, as the outer sheath slides over the stent device, drag force between the outer sheath and the stent device is translated to axial forces on the stent device that is held by the stent device pusher.

WO 96/39998 discloses a delivery catheter where the inner catheter has a stop mounted on it axially adjacent a proximal end of the stent device. The stop serves as a stent device pusher, preventing axial movement in the proximal direction as the outer sheath is retracted. For short, axially strong stents, this design is fine. A particular application may require longer stent devices that are desirably flexible so that the tortuous passageways of the vascular system can be traversed. Flexibility and axial strength present a trade-off in properties, where a more flexible stent device is an axially less strong one. In designs of delivery catheters where a stop acts on a proximal end of the stent device, there is a greater risk with more flexible stents of the stent device deforming in the longitudinal direction as friction between the outer sheath and the stent induces axial forces that are focused at the proximal end of the stent.

International patent publication number WO0071058 recognised this problem with low column strength stent devices being axially held on an inner catheter at the proximal end of the stent device. The document discloses to use a stabiliser disposed within the interior of the stent device that is adapted to engage the inner periphery of the stent device. The surface element may be a high friction surface or a plurality of protuberances engaged with the inner surface of the stent device. In one example, the inner catheter may be coated with a pressure-sensitive adhesive. The stabilizer serves to transmit longitudinal force to the stent device, and displaces the stent device relative to the outer sheath without collapsing the low column strength stent device.

International patent publication number WO 2004/096091 discloses a stent device delivery system that aims to distribute axial forces along the stent device during loading of a self-expanding stent device into an outer sheath or deployment of the stent device out of the outer sheath. This is achieved by the provision of an inner catheter with protrusions and recesses along an outer surface, where the protrusions are embedded within an inner cover layer, made of expanded polytetrafluouroethylene (ePTFE), of a stent device. The embedded protrusions provide a "form fit" between the stent device and the stent device pusher, which means that as the outer sheath is moved relative to the stent device, resultant axial forces on the stent device are effectively distributed along it.

As stent devices become more flexible the relevance increases of uniformly distributing axial forces along and about the stent device during loading and deployment of the stent device.

It is, therefore, an object of the present invention to provide a stent device delivery system and a method of loading the stent device to the delivery system, whereby axial forces caused by an outer sheath moving relative to a stent device axially held on an inner catheter are effectively distributed uniformly along and about the stent device.

### Summary of the Invention

The invention is defined by the independent claims.
In view of the above object, a first aspect of the present invention provides a stent device delivery system, comprising a stent device pusher, a radially self-expandable stent device and an outer sheath. The stent device comprises a lumen and defines a longitudinal axis. The stent device pusher comprises protrusions with axially extending spaces between them. The stent device is constrained in a radially contracted state by the outer sheath so that the protrusions engage an inner surface of the stent device at positions axially distributed along the stent device. The protrusions thus provide a bed for the stent device. The delivery system is characterised by a tacky material axially distributed along the stent device providing tackiness between the stent device pusher and the inner surface of the stent device. The tackiness and the protrusions serve to resist axial movement of the stent device relative to the stent device pusher as the outer sheath slides axially over the stent device. The tacky material is such that it allows the stent device to peel from it and expand to a radially expanded state as the outer sheath slides axially over the stent device to unconstrain the stent device.
A method of loading the stent device into an outer sheath of a stent device delivery system is also provided. The method comprises providing a radially self-expandable stent device having a lumen and defining a longitudinal axis. The method also includes providing a stent device pusher comprising protrusions with axially extending spaces between them. The method includes constraining the stent device within the outer sheath so that an inner surface of the stent device engages the protrusions at positions distributed axially along the stent device. The protrusions provide a bed for the stent device. The method is characterised by providing tackiness between the inner surface of the stent device and the stent device pusher with a tacky material. The tacky material is distributed along the stent device. The tackiness and the protrusions serve to resist axial movement of the stent device relative to the stent device pusher as the outer sheath slides over the stent device. The tacky material is so as to allow the stent device to peel from it to expand to a radially expanded state when the outer sheath is retracted and the stent device is thereby unconstrained.

In both the first and second aspects of the present invention, the protrusions or protruding portions allow a degree of form fit with the stent device. This mixture of form fit and tackiness has been found to be particularly effective in allowing uniform distribution of the compressive force along the stent device as the outer sheath is dragged over the stent device's outer surface. Further, the tackiness will provide some force holding the stent device in the radially contracted state, which serves to reduce the strain on the outer sheath.

The protrusions allow a form fit between the stent device pusher and the stent device serving to resist axial movement of the stent device relative to the stent device pusher by deforming the stent device such that portions of the stent device extend radially inwardly axially between the protrusions.

Engagement between the sides of the protrusions and the portions of the stent device between (along an axial line between the protrusions) them can provide a form fitting axial lock, while the tackiness makes use of the outer surface of the protrusion for resisting axial movement of the stent device relative to the stent device pusher. This mixture of form fitting protrusions and tackiness has been found to allow uniform distribution of the compressive force along the stent device as the outer sheath is dragged over the stent device's outer surface.

In a preferred embodiment, the first and second aspects of the present invention are combined so that the protrusions of the first aspect are compressible and the stent device is embedded in the compressible protrusions so that portions of the compressible protrusions protrude into the stent device at positions distributed axially along the stent device or the compressible bed of the second aspect forms protrusions with axially extending spaces between them.

The protrusions are, by the restraining force of the outer sheath, compressed between the stent device and an inner catheter of the stent device pusher. Thus, the amount of protrusions that are compressed contribute to the crimping force required to load a stent device into an outer sheath and the amount of radial strength the outer sheath needs to restrain the stent device. It can, therefore, be advantageous to reduce the amount of protrusions distributed along the stent device, but a sufficient axial hold on the stent device during retraction of the outer sheath must also be provided. The combination of protrusions and tackiness allows such reduction in the number of protrusions because the tackiness contributes to axial resistance.
A number of types of tacky materials can be selected and distributed along the stent device in a number of ways with the aim of distributing the axial forces caused by drag as the outer sheath slides over the stent device, while at the same time ensuring that the tacky material is not so tacky as to prevent the stent device from expanding into its radially enlarged state during deployment.

The tacky material offers a degree of radial force holding the stent device to the stent device pusher and thus offers some resistance to radial expansion of the stent device, requiring the stent device to peel away from it during radial expansion. The tacky material may be a rubber material, a silicone adhesive or an adhesive based on polyether block amides, such as the one sold under the trade name PEBAX. The presently most preferred material is PEBAX 3533. PEBAX 3533 is an extremely soft material (about 35 shore durometer) that has been found to have the right amount of tackiness. The tacky material is an adhesive material in a preferred embodiment, adhesively bonding the stent device pusher to the inner surface of the stent device. The tacky material may be one that can be activated by exposure to radiation for flexibility in manufacturing.

The invention has the tacky material in a helix about the longitudinal axis of the stent device, the helix extending along the length of the inner surface of the stent device. Such a uniform configuration also facilitates uniformly distributing the axial forces along and about the stent device as the sheath is retracted over the stent device during deployment. This configuration may also be advantageous as it allows a relatively strong tackiness to be used for the purpose of fixing the axial position of the stent device to the stent device pusher, while at the same time offering a sufficiently low radial peel force for the purpose of allowing the stent device to expand as the outer sheath is retracted during deployment.

In a preferred embdodiment, there is tackiness between the stent device bed and the inner surface of the stent device by way of the tacky material.

Preferably, the protrusions allow a form fit between the stent device pusher and the stent device serving to resist axial movement of the stent device relative to the stent device pusher by deforming the stent device such that portions of the stent device extend radially inwardly axially between the protrusions.

Engagement between the sides of the protrusions and the portions of the stent device between (along an axial line between the protrusions) them can provide a form fitting axial lock, while the tackiness makes use of the outer surface of the protrusion for resisting axial movement of the stent device relative to the stent device pusher. This mixture of form fitting protrusions and tackiness has been found to allow uniform distribution of the compressive force along the stent device as the outer sheath is dragged over the stent device's outer surface.

The stent device includes a tubular frame for resisting radial forces to support and keep open a vascular portion and an inner cover, such as an ePTFE layer, providing a layer covering an inner surface of the tubular frame. The tubular frame may be in the form of a mesh and so have apertures through the frame. The tubular frame is preferably made of Nitinol. The inner cover may provide a liquid impermeable inner wall to the stent device. In this case, the tacky material adheres to the inner cover. The protrusions deform the inner cover to provide some form fit with the stent device.

Preferably, the tacky material is an adhesive material in that it adhesively bonds to an inner catheter of the stent device pusher. In this way, the tacky material adhesively secures to the inner catheter and also provides tackiness to the inner surface of the stent device.

The tacky material may be radially compressed into a deformed state between the outer sheath and the stent device where the tacky material is resiliently biased radially outwardly.
This feature of the tacky material ensures a tight fit between the inner catheter and the stent device so as to ensure strong engagement of the protrusions with the stent device and thus sufficient resistance to axial movement of the stent device relative to the stent device pusher.

The protrusions are in the form of a helix extending along the length of the stent device. In an alternative, the protrusions are in the form of helices distributed along the stent device with spaces between them. Taking a cross-section of the stent device pusher along the longitudinal axis will reveal how a helix can be considered to give protrusions separated with an axial space between them even though the helix is just one continuous protrusion. The pitch of the helix will determine the space between adjacent protrusions along an axial line.

The pitch of the helix or the density of the bands or helices as well as the height of the protrusions in a radial direction can be determined based on the degree of engagement with the stent device that is needed, while taking into consideration the desire to reduce the crimping force for compressing the protrusions and the resulting restoration force on the outer sheath and results that prove optimal for a particular application.

The protrusions are made of some other material with tacky material applied to it for adhering to the inner surface of the stent device. In this latter case, and in the instance of protrusions, the tacky material is also used to secure the non-tacky material to an inner catheter of the stent pusher. Forming the protrusions of the tacky material may be advantageous for reducing manufacturing steps, but other materials may offer greater design flexibility. In particular, the tacky material may be compressible to deform under the constraining force of the outer sheath to provide the tight fit between the stent pusher and the stent device, which means that the non-tacky material is not required to be deformed by the constraining force of the outer sheath.

Preferred embodiments of the present invention will be described in the following with reference to the figures.

### Brief Description of the Figures

Figure 1 shows a first example of a system of a stent device with an inner cover and a stent device pusher.
Figure 2 shows an embodiment of the stent device pusher.
Figure 3 shows a second example.

### Detailed Description of Preferred Examples and Embodiments

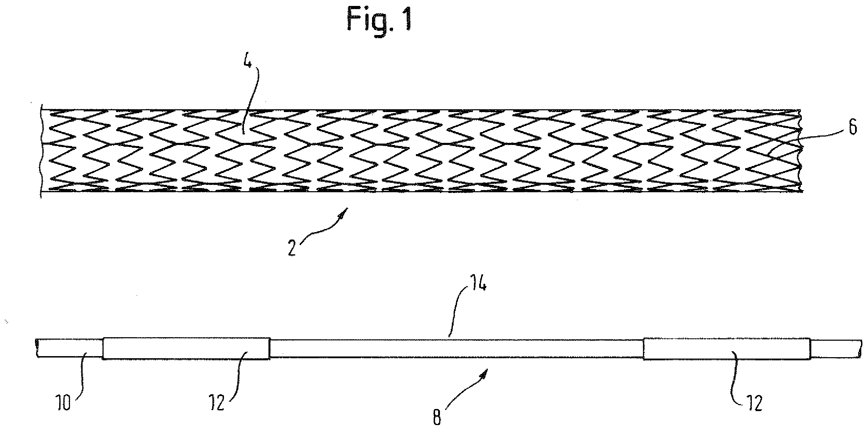

A tubular, radially expandable, stent device 2 is shown in figure 1 in a radially expanded state. The stent device 2 is collapsible into a radially contracted state (not shown). The stent device 2 includes a mesh structure 6 for providing a framework to support a vascular lumen and also an inner cover 4 defining an inner surface of the tubular stent device 2 for providing a continuous surface through which bodily fluid can travel. The mesh structure 6 is preferably made of Nitinol and the cover material 4 is preferably made of ePTFE. Other suitable materials for the mesh structure and the cover can be used. Similarly, other cover materials known in the art can also be used. An outer cover material layer of ePTFE (not shown) may also be provided. The mesh structure 6 comprises a number of zigzag stenting turns extending about the lumen and centered on the longitudinal axis of the stent device. The stenting turns are connected axially by bridging elements.
Also shown in figure 1 is a stent device pusher 8. The stent device pusher 8 comprises an inner catheter 10 and a plurality of axially distributed protrusions in the form of elongate bands 12 mounted on the inner catheter 10. The inner catheter 10 comprises a reinforced polyimide tube that is braided with stainless steel. The braiding provides axial strength to the inner catheter 10. There are two bands 12 shown in the figure that are separated axially to provide a space 14 therebetween. The bands 12 in this example are the axial length of about five stenting turns, while the space 14 between adjacent bands 12 is about ten stenting turns. The full length of the stent device 2 and the stent device pusher 8 cannot be seen in figure 1, but it is to be understood that the bands 12 are distributed along the entire length of the stent device 2. The bands 12 are made of a material that is compressible and an adhesive, more particularly PEBAX 3533. Alternatively, the bands 12 could be made by spraying rubber on the inner catheter 10 and allowing it to harden. In another example, the bands 12 are made by applying silicone glue to the inner catheter 10 and hardening it by application of ultraviolet light. In each of these cases, the material of the bands 12 is applied to the inner catheter 10 and bonds with the inner catheter 10. The bands 12 present a tacky surface to the inner surface of the stent device 2.

In order to load the stent device 2 onto the stent device pusher 8, the stent device 2 is crimped into its radially contracted state about the stent device pusher 8 so that the stent device pusher 8 extends through the lumen of the stent 6, from one end to the opposite end. The bands 12 will protrude into the inner cover material 4 of the stent device 2 and the inner cover material 4 will protrude into the axial space 14 between the bands 12, thereby providing a form fit. Further, the stent device 2 will be embedded in the bands 12, causing portions of the bands 12 to protrude radially into the stent device 2. This latter effect may be particularly prevalent in bare stent applications. Although there is embedding, the stent device 2 still defines a tubular outer periphery that is radially outward of a tubular outer periphery of the bands 12. Radially extending edges of the inner cover material 4 and the bands 12 will result where the bands begin and end. Further, embedding of the stent device 2 into the bands 12 will cause radially protruding portions of the bands 12 to have radially extending edges engaging radially extending edges of the stent device 2. These interfacing edges provide an axial lock serving to resist relative axial movement between the stent device pusher 8 and the stent device 2. The material of the bands 12 adhesively bonds the bands 12 to the inner catheter 14. The adhesive material also serves to present a tacky surface to the inner surface of the stent device 2 to resist relative axial movement between the stent device 2 and the stent device pusher 8.

The stent device pusher 8, with the contracted stent device 2 mounted on it, is loaded into an outer sheath (not shown) of a stent device delivery system. The outer sheath serves to maintain the stent device 2 in its contracted state while the stent device 2 is delivered to a vascular lumen. The bands 12 are deformed into a compressed configuration by the crimping force of the stent when it is mounted about the stent device pusher 8 and this radially inward force is maintained by the outer sheath. The adhesive material of the bands 12 is resilient in nature and thus provides a radially outward force tending to restore the bands 12 from their compressed configuration. This thus forces the bands 12 into the inner surface of the stent device 2, thereby reinforcing the form fit and the tacky interaction between the stent device 2 and the pusher 8.

The outer sheath slides over the stent device 2 as the stent device 2 is loaded into the outer sheath. The relative position of the stent device pusher 8 and the stent device 2 is maintained as the outer sheath slides over the stent device 2 by the tackiness and the form fit therebetween. The drag of the outer sheath over the stent device 2 causes axial forces on the stent device 2. The mixture of form fit and tackiness has been found to provide a particularly effective way of facilitating distributing of these forces uniformly along the stent device 2 during loading of the stent device 2 into the outer sheath.

A stent device delivery system will be used to position the stent device 2 mounted on the stent device pusher 8 at the desired location along a vascular lumen. The outer sheath is then retracted, which unconstrains the stent device 2 allowing the stent device 2 to progressively, in the axial direction, expand to the expanded state and engage and support the inner wall of the vascular lumen. As the stent device 2 expands, it peels away from the tacky interaction and thus dismounts from the stent device pusher 8. In the expanded state, the form fit with the inner surface of the stent device 2 and the tacky interaction is lost, leaving the stent pusher 8 free to be removed from within the stent device 2 and removed from the body.

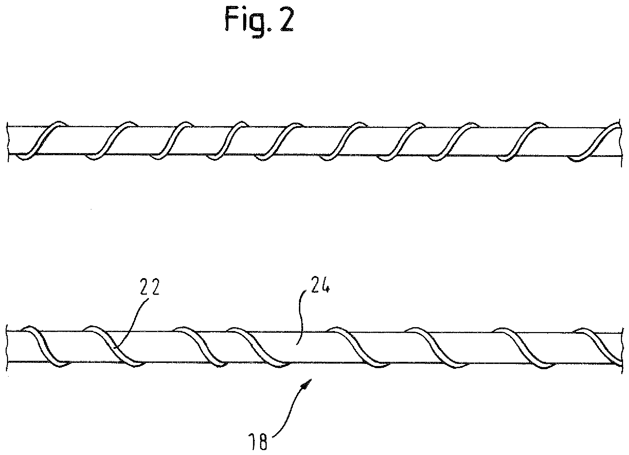

Figure 2 shows the stent device pusher according to a preferred embodiment of the invention. The stent device pusher 18 comprises a braided inner catheter 24, as with the stent device pusher of figure 1, but, instead of protrusions in the form of bands as in the embodiment of figure 1, a plurality of axially distributed protrusions 22 are provided by a helically arranged wire mounted on the inner catheter 24.

The wire is helically wrapped about the inner catheter 24 and adhesively bonded to the inner catheter 24 with an adhesive material. The wire and the adhesive material form axially distributed protrusions 22. The adhesive material also serves to provide a tacky interaction between the protrusion 22 and an inner surface, which is preferably an inner cover of for example ePTFE, of a stent device. The adhesive is preferably PEBAX 3533, which has been found to have the right amount of tackiness. Alternative materials such as silicone adhesive or rubber as described above may be used, provided they offer a radial holding force from which the stent device peels from during expansion.

Only a part of the pusher element 8 is shown in figure 2. In one embodiment, the helical wire 22 extends the full length of the stent device. In another embodiment, a plurality of helices is provided that are axially spaced from one another and the distribution of these helices extends the full length of the stent device. The pitch of the helix or each of the helices defines an axial space between adjacent protrusions 22 along an axial line passing through the protrusions 22.

When the stent device is mounted on the stent device pusher 18, the protrusion 22 embeds within an inner cover of the stent device providing a form fit that serves to resist relative axial movement between the stent device pusher 18 and the stent device. Adhesive material is provided between the inner catheter 24 and the wire to bond them together. The inner cover of the stent device will deform about the protrusion 22 when the stent device is crimped onto the stent device pusher 18, causing the inner cover to be in contact with the adhesive material bonding the protrusion to the inner catheter 14. Accordingly, adhesive material does not necessarily need to be provided on the radial top of the wire. Instead, the inner cover may access the adhesive material from either side of the wire 22 by wrapping the inner cover about the wire 22.

During delivery of the stent device, as the outer sheath is retracted and the stent device is able to expand, the stent device peels away from the tacky interaction and thus dismounts from the stent device pusher 18.

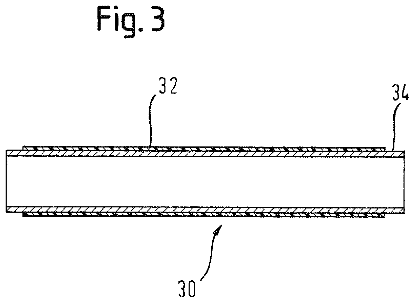

Figure 3 shows a second example. In this example, a tacky material layer 32 is disposed continuously along and about the inner catheter 34 to form a bed for receiving a stent device. The tacky material is compressible so that the stent device can be embedded in the layer 32. Some portions of the layer 32 will be relatively compressed by the stent device, e.g. portions constrained by the tubular frame of the stent device, and other portions of the layer 32 will protrude radially into the stent device, e.g. where spaces in the tubular frame of the stent device allow room for the protrusions.

The layer 21 is preferably sprayed on the inner catheter 34 and bonds to the inner catheter 34. Preferably, the layer 21 is made from a rubber material. Alternatively, the layer is made from a silicone glue material or is a polyether block amide based adhesive, such as the one sold under the trade name PEBAX 3533. These materials provide a tacky interaction with the inner surface of the stent device, which the stent device peels away from during radial expansion for deployment.

The thickness of the layer of tacky material 32 or the protrusions 12, 22 made of the tacky material may be in the range of 0.05 mm to 0.25 mm, for example.

In alternative forms to the embodiment shown in figure 2, the helically arranged wire 22 may be replaced by helically arranged tacky material to provide a plurality of axially distributed protrusions. In this embodiment, the helically arranged material is preferably compressible and provides a tacky interaction with the inner surface of the stent device. The compressibility of the material means that some radial portions of the helix will be compressed, e.g. where the stent rings are, while other portions of the helix will protrude into the stent device, e.g. into spaces in the tubular frame of the stent device. Thus, even when a bare stent is used, there is a form fit between the stent device pusher and the stent device. The tacky material may be resilient so that compressed portions tend to revert to at least substantially their uncompressed configurations.

## Claims

1. A stent device delivery system, comprising:
a stent device pusher (8) comprising an inner catheter,
a radially self-expandable stent device (2) and
an outer sheath,
the stent device comprising a lumen extending longitudinally therethrough,
the stent device pusher comprising protrusions (12, 22) with axially extending spaces (14, 24) between them,
the stent device being constrained in a radially contracted state by the outer sheath so that the protrusions of the stent pusher engage an inner surface of the stent device at positions axially distributed along the stent device,
wherein the protrusions deform the stent device such that portions of the stent device extend radially inwardly axially between the protrusions to provide a form fit between the stent device pusher and the stent device,
wherein the stent device comprises an inner cover (4) providing a layer covering an inner surface of a tubular frame (6) of the stent device,
and wherein the protrusions deform the inner cover to provide a form fit between the stent device and the stent device pusher;
**characterised by** a tacky material axially distributed along the stent device and providing a tackiness between the stent device pusher and the inner surface of the stent device,
wherein the tackiness and the engagement of the protrusions with the inner surface of the stent device serve to resist axial movement of the stent device relative to the stent device pusher as the outer sheath slides axially over the stent device and the tacky material is such that it allows the stent device to peel from the tacky material and expand to a radially expanded state as the outer sheath slides axially over the stent device to unconstrain the stent device,
wherein the tacky material provides a tacky interaction with the inner cover, and
wherein the protrusions form a tacky interaction with the inner surface of the stent device by way of the tacky material,
wherein the protrusions form a helix extending along the length of the stent device, the helix being formed by a wire being helically wrapped about the inner catheter and being adhesively bonded to the inner catheter with an adhesive material, the adhesive material also serving to provide a tacky interaction between the protrusions and an inner surface of the stent device.

2. The stent device delivery system of claim 1, wherein the tacky material is uniformly distributed along and about the inner surface of the stent device.

3. The stent device delivery system of any one of the preceding claims, wherein the tacky material is a rubber material or an adhesive material, preferably a silicone adhesive material or a polyether block amide based adhesive material.

4. The stent device delivery system of any one of the preceding claims, wherein the tubular frame is for resisting radial forces to maintain a vascular portion open.

5. The stent device delivery system of any one of the preceding claims, wherein the stent device pusher comprises an inner catheter (10) having the protrusions mounted thereon.

6. The stent device delivery system of claim 5, wherein the tacky material is located such that the protrusions provide a tacky interaction with the inner surface of the stent device and further, the tacky material is so that the protrusions are bonded to the inner catheter.

7. The stent delivery device of claim 5 or 6, wherein the tacky material is deformed into a compressed state by the stent device being embedded therein, whereby portions of the tacky material protrude radially into the stent device at positions axially distributed along the stent device to provide a form fit between the stent device pusher and the stent device.

8. The stent delivery system of any one of the preceding claims, wherein the plurality of protrusions form a compressible bed, whereby the stent device is embedded in the compressible bed so that portions of the compressible bed protrude radially into the stent device at positions axially distributed along the stent device.

9. The stent device delivery system of any one of the preceding claims, wherein the protrusions are formed of the tacky material.

10. A method of loading a stent device (2) into an outer sheath of a stent device delivery system, comprising
providing a radially self-expandable stent device (2) having a lumen extending longitudinally therethrough,
providing a stent device pusher (8) comprising protrusions (12, 22) with axially extending spaces between them, the stent device pusher (8) further comprising an inner catheter,
inserting the stent device mounted to the stent device pusher into the outer sheath of the stent device delivery system so that the stent device is constrained within the outer sheath and an inner surface of the stent device engages the protrusions at positions distributed axially along the stent device,
wherein the protrusions deform the stent device such that portions of the stent device extend radially inwardly axially between the protrusions to provide a form fit between the stent device pusher and the stent device,
wherein the stent device comprises an inner cover (4) providing a layer covering an inner surface of a tubular frame (6) of the stent device, and
wherein the protrusions deform the inner cover to provide a form fit between the stent device and the stent device pusher;
**characterised by** an adhesive material distributed along the stent device providing a tackiness between the inner surface of the stent device and the stent device pusher,
wherein the tackiness and the engagement of the protrusions with the inner surface of the stent device serves to resist axial movement of the stent device relative to the stent device pusher as the outer sheath slides over the stent device and the tacky material is such that it allows the stent device to peel from the tacky material to expand to a radially expanded state when the outer sheath is retracted and the stent device is thereby unconstrained,
wherein the tacky material provides a tacky interaction with the inner cover, and
wherein the protrusions form a tacky interaction with the inner surface of the stent device by way of the tacky material,
wherein the protrusions form a helix extending along the length of the stent device the helix being formed by a wire being helically wrapped about the inner catheter and being adhesively bonded to the inner catheter with an adhesive material, the adhesive material also serving to provide a tacky interaction between the protrusions and an inner surface of the stent device.

## Patentansprüche

1. Stentvorrichtung-Freisetzungssystem, umfassend:
ein Stentvorrichtung-Schiebeelement (8), umfassend einen Innenkatheter,
eine radial selbstexpandierende Stentvorrichtung (2) und
eine Außenhülle,
wobei die Stentvorrichtung einen sich längs durch sie hindurch erstreckenden Hohlraum umfasst,
wobei das Stentvorrichtung-Schiebeelement Vorsprünge (12, 22) mit sich axial dazwischen erstreckenden Zwischenräumen (14, 24) umfasst,
wobei die Stentvorrichtung von der Außenhülle derart in einem radial zusammengezogenen Zustand eingeschnürt ist, dass die Vorsprünge des Stentschiebeelements an axial verteilten Stellen entlang der Stentvorrichtung in eine Innenfläche der Stentvorrichtung eingreifen,
wobei die Vorsprünge die Stentvorrichtung derart verformen, dass sich Abschnitte der Stentvorrichtung axial zwischen den Vorsprüngen radial so nach innen erstrecken, dass sie für einen Formschluss zwischen dem Stentvorrichtung-Schiebeelement und der Stentvorrichtung sorgen,
wobei die Stentvorrichtung einen Innenüberzug (4) umfasst, der eine Schicht bereitstellt, die eine Innenfläche eines röhrenförmigen Gerüsts (6) der Stentvorrichtung bedeckt,
und wobei die Vorsprünge den Innenüberzug verformen und so für einen Formschluss zwischen der Stentvorrichtung und dem Stentvorrichtung-Schiebeelement sorgen;
**gekennzeichnet durch** ein haftendes Material, das axial die Stentvorrichtung entlang verteilt ist und für eine Haftbindung zwischen dem Stentvorrichtung-Schiebeelement und der Innenfläche der Stentvorrichtung sorgt,
wobei die Haftbindung und der Eingriff der Vorsprünge in die Innenfläche der Stentvorrichtung dazu dienen, einer axialen Bewegung der Stentvorrichtung bezogen auf das Stentvorrichtung-Schiebeelement standzuhalten, wenn die Außenhülle axial über die Stentvorrichtung gleitet, und das haftende Material derart ist, dass sich die Stentvorrichtung von dem haftenden Material ablösen und sich in einen radial aufgeweiteten Zustand aufweiten kann, wenn die Außenhülle axial über die Stentvorrichtung gleitet und so die Stentvorrichtung freigibt,
wobei das haftende Material für eine Haftwechselwirkung mit dem Innenüberzug sorgt, und
wobei die Vorsprünge mit der Innenfläche der Stentvorrichtung über das haftende Material eine haftende Wechselwirkung eingehen,
wobei die Vorsprünge eine sich die Länge der Stentvorrichtung entlang erstreckende Spirale bilden, wobei die Spirale von einem spiralförmig um den Innenkatheter gewickelten Draht gebildet ist, der mit einem Klebstoffmaterial mit dem Innenkatheter verklebt ist, wobei das Klebstoffmaterial auch dazu dient, für eine Haftwechselwirkung zwischen den Vorsprüngen und einer Innenfläche der Stentvorrichtung zu sorgen.

2. Stentvorrichtung-Freisetzungssystem nach Anspruch 1, wobei das haftende Material gleichmäßig entlang der und um die Innenfläche der Stentvorrichtung herum verteilt ist.

3. Stentvorrichtung-Freisetzungssystem nach einem der vorstehenden Ansprüche, wobei das haftende Material ein Kautschukmaterial oder ein Klebstoffmaterial ist, vorzugsweise ein Silikonklebstoffmaterial oder ein Klebstoffmaterial auf Basis von Polyetherblockamiden.

4. Stentvorrichtung-Freisetzungssystem nach einem der vorstehenden Ansprüche, wobei das röhrenförmige Gerüst dazu dient, Radialkräften standzuhalten und so einen Gefäßabschnitt offenzuhalten.

5. Stentvorrichtung-Freisetzungssystem nach einem der vorstehenden Ansprüche, wobei das Stentvorrichtung-Schiebeelement einen Innenkatheter (10) umfasst, an dem die Vorsprünge befestigt sind.

6. Stentvorrichtung-Freisetzungssystem nach Anspruch 5, wobei das haftende Material derart angeordnet ist, dass die Vorsprünge für eine Haftwechselwirkung mit der Innenfläche der Stentvorrichtung sorgen und das haftende Material weiter derart ist, dass die Vorsprünge an dem Innenkatheter angeklebt sind.

7. Stent-Freisetzungsvorrichtung nach Anspruch 5 oder 6, wobei das haftende Material von der darin eingebetteten Stentvorrichtung in einen zusammengedrückten Zustand verformt wird, wodurch Abschnitte des haftenden Materials an axial entlang der Stentvorrichtung verteilten Stellen radial in die Stentvorrichtung ragen und so für einen Formschluss zwischen dem Stentvorrichtung-Schiebeelement und der Stentvorrichtung sorgen.

8. Stent-Freisetzungssystem nach einem der vorstehenden Ansprüche, wobei die mehreren Vorsprünge ein zusammendrückbares Bett bilden, wodurch die Stentvorrichtung in dem zusammendrückbaren Bett derart eingebettet ist, dass Abschnitte des zusammendrückbaren Betts an axial entlang der Stentvorrichtung verteilten Stellen radial in die Stentvorrichtung ragen.

9. Stentvorrichtung-Freisetzungssystem nach einem der vorstehenden Ansprüche, wobei die Vorsprünge aus dem haftenden Material gebildet sind.

10. Verfahren zum Laden einer Stentvorrichtung (2) in eine Außenhülle eines Stentvorrichtung-Freisetzungssystems, umfassend
Bereitstellen einer radial selbstexpandierbaren Stentvorrichtung (2), die einensich längs durch sie hindurch erstreckenden Hohlraum aufweist,
Bereitstellen eines Stentvorrichtung-Schiebeelements (8), das Vorsprünge (12, 22) mit sich axial dazwischen erstreckenden Zwischenräumen umfasst, wobei das Stentvorrichtung-Schiebeelement (8) weiter einen Innenkatheter umfasst,
Einführen der am Stentvorrichtung-Schiebeelement befestigten Stentvorrichtung in die Außenhülle des Stentvorrichtung-Freisetzungssystems, sodass die Stentvorrichtung in der Außenhülle eingeschnürt ist und eine Innenfläche der Stentvorrichtung an axial entlang der Stentvorrichtung verteilten Stellen mit den Vorsprüngen in Eingriff gelangt,
wobei die Vorsprünge die Stentvorrichtung derart verformen, dass sich Abschnitte der Stentvorrichtung axial zwischen den Vorsprüngen radial so nach innen erstrecken, dass sie für einen Formschluss zwischen dem Stentvorrichtung-Schiebeelement und der Stentvorrichtung sorgen,
wobei die Stentvorrichtung einen Innenüberzug (4) umfasst, der eine Schicht bereitstellt, die eine Innenfläche eines röhrenförmigen Gerüsts (6) der Stentvorrichtung bedeckt, und
wobei die Vorsprünge den Innenüberzug verformen und so für einen Formschluss zwischen der Stentvorrichtung und dem Stentvorrichtung-Schiebeelement sorgen;
**gekennzeichnet durch** ein Klebstoffmaterial, das die Stentvorrichtung entlang verteilt ist und für eine Haftbindung zwischen der Innenfläche der Stentvorrichtung und dem Stentvorrichtung-Schiebeelement sorgt,
wobei die Haftbindung und der Eingriff der Vorsprünge in die Innenfläche der Stentvorrichtung dazu dienen, einer axialen Bewegung der Stentvorrichtung bezogen auf das Stentvorrichtung-Schiebeelement standzuhalten, wenn die Außenhülle über die Stentvorrichtung gleitet, und das haftende Material derart ist, dass sich die Stentvorrichtung von dem haftenden Material ablösen und sich so in einen radial aufgeweiteten Zustand aufweiten kann, wenn die Außenhülle zurückgezogen wird und die Stentvorrichtung dadurch freigeben wird,
wobei das haftende Material für eine Haftwechselwirkung mit dem Innenüberzug sorgt, und
wobei die Vorsprünge mit der Innenfläche der Stentvorrichtung über das haftende Material eine haftende Wechselwirkung eingehen,
wobei die Vorsprünge eine sich die Länge der Stentvorrichtung entlang erstreckende Spirale bilden, wobei die Spirale von einem spiralförmig um den Innenkatheter gewickelten Draht gebildet ist, der mit einem Klebstoffmaterial mit dem Innenkatheter verklebt ist, wobei das Klebstoffmaterial auch dazu dient, für eine Haftwechselwirkung zwischen den Vorsprüngen und einer Innenfläche der Stentvorrichtung zu sorgen.

## Revendications

1. Système de pose d'un dispositif stent, comprenant :
un poussoir de dispositif stent (8) comprenant un cathéter interne,
un dispositif stent radialement autodéployable (2) et
une gaine externe,
le dispositif stent comprenant une lumière s'étendant longitudinalement à travers celui-ci,
le poussoir de dispositif stent comprenant des saillies (12, 22) avec des espaces s'étendant axialement (14, 24) entre elles,
le dispositif stent étant contraint dans un état radialement contracté par la gaine externe de sorte que les saillies du poussoir de stent viennent en prise avec une surface interne du dispositif stent en des positions réparties axialement le long du dispositif stent,
dans lequel les saillies déforment le dispositif stent de sorte que des portions du dispositif stent s'étendent radialement vers l'intérieur axialement entre les saillies pour fournir une forme adaptée entre le poussoir de dispositif stent et le dispositif stent,
dans lequel le dispositif stent comprend un revêtement interne (4) fournissant une couche recouvrant une surface interne d'une structure tubulaire (6) du dispositif stent,
et dans lequel les saillies déforment le revêtement interne pour fournir une forme adaptée entre le dispositif stent et le poussoir de dispositif stent ;
**caractérisé par** un matériau collant réparti axialement le long du dispositif stent et fournissant une texture collante entre le poussoir de dispositif stent et la surface interne du dispositif stent,
dans lequel la texture collante et la mise en prise des saillies avec la surface interne du dispositif stent servent à résister à un mouvement axial du dispositif stent par rapport au poussoir de dispositif stent à mesure que la gaine externe glisse axialement sur le dispositif stent et le matériau collant est tel qu'il permet au dispositif stent de se décoller du matériau collant et de se déployer dans un état radialement déployé à mesure que la gaine externe glisse axialement sur le dispositif stent pour libérer le dispositif stent,
dans lequel le matériau collant fournit une interaction collante avec le revêtement interne, et
dans lequel les saillies forment une interaction collante avec la surface interne du dispositif stent par le biais du matériau collant,
dans lequel les saillies forment une spirale s'étendant sur la longueur du dispositif stent, la spirale étant formée par un fil étant enroulé en spirale autour du cathéter interne et étant lié de manière adhésive au cathéter interne avec un matériau adhésif, le matériau adhésif servant également à fournir une interaction collante entre les saillies et une surface interne du dispositif stent.

2. Système de pose de dispositif stent selon la revendication 1, dans lequel le matériau collant est réparti uniformément le long et autour de la surface interne du dispositif stent.

3. Système de pose de dispositif stent selon l'une quelconque des revendications précédentes, dans lequel le matériau collant est un matériau en caoutchouc ou un matériau adhésif, de préférence un matériau adhésif en silicone ou un matériau adhésif à base de polyéther bloc amide.

4. Système de pose de dispositif stent selon l'une quelconque des revendications précédentes, dans lequel la structure tubulaire est destinée à résister à des forces radiales pour maintenir une portion vasculaire ouverte.

5. Système de pose de dispositif stent selon l'une quelconque des revendications précédentes, dans lequel le poussoir de dispositif stent comprend un cathéter interne (10) présentant les saillies montées sur celui-ci.

6. Système de pose de dispositif stent selon la revendication 5, dans lequel le matériau collant est situé de sorte que les saillies fournissent une interaction collante avec la surface interne du dispositif stent et en outre, le matériau collant est tel que les saillies sont liées au cathéter interne.

7. Dispositif de pose de stent selon la revendication 5 ou 6, dans lequel le matériau collant est déformé en un état comprimé par le dispositif stent étant intégré dans celui-ci, moyennant quoi des portions du matériau collant font saillie radialement dans le dispositif stent en des positions réparties axialement le long du dispositif stent pour fournir une forme adaptée entre le poussoir de dispositif stent et le dispositif stent.

8. Système de pose de stent selon l'une quelconque des revendications précédentes, dans lequel la pluralité de saillies forment un lit comprimable, moyennant quoi le dispositif stent est intégré dans le lit comprimable de sorte que des portions du lit comprimable fassent saillie radialement dans le dispositif stent en des positions réparties axialement le long du dispositif stent.

9. Système de pose de dispositif stent selon l'une quelconque des revendications précédentes, dans lequel les saillies sont composées du matériau collant.

10. Procédé d'introduction d'un dispositif stent (2) dans une gaine externe d'un système de pose de dispositif stent, comprenant
la fourniture d'un dispositif stent radialement autodéployable (2) présentant une lumière s'étendant de manière longitudinale à travers celui-ci,
la fourniture d'un poussoir de dispositif stent (8) comprenant des saillies (12, 22) avec des espaces s'étendant axialement entre elles, le poussoir de dispositif stent (8) comprenant en outre un cathéter interne,
l'insertion du dispositif stent monté sur le poussoir de dispositif stent dans la gaine externe du système de pose de dispositif stent de sorte que le dispositif stent soit contraint à l'intérieur de la gaine externe et qu'une surface interne du dispositif stent vienne en prise avec les saillies en des positions réparties axialement le long du dispositif stent,
dans lequel les saillies déforment le dispositif stent de sorte que des portions du dispositif stent s'étendent radialement vers l'intérieur axialement entre les saillies pour fournir une forme adaptée entre le poussoir de dispositif stent et le dispositif stent,
dans lequel le dispositif stent comprend un revêtement interne (4) fournissant une couche recouvrant une surface interne d'une structure tubulaire (6) du dispositif stent, et
dans lequel les saillies déforment le revêtement interne pour fournir une forme adaptée entre le dispositif stent et le poussoir de dispositif stent ;
**caractérisé par** un matériau adhésif réparti le long du dispositif stent fournissant une texture collante entre la surface interne du dispositif stent et le poussoir de dispositif stent,
dans lequel la texture collante et la mise en prise des saillies avec la surface interne du dispositif stent servent à résister à un mouvement axial du dispositif stent par rapport au poussoir de dispositif stent à mesure que la gaine externe glisse sur le dispositif stent et le matériau collant est tel qu'il permet au dispositif stent de se décoller du matériau collant pour se déployer dans un état radialement déployé lorsque la gaine externe est rétractée et le dispositif stent est ainsi libéré,
dans lequel le matériau collant fournit une interaction collante avec le revêtement interne, et
dans lequel les saillies forment une interaction collante avec la surface interne du dispositif stent par le biais du matériau collant,
dans lequel les saillies forment une spirale s'étendant sur la longueur du dispositif stent, la spirale étant formée par un fil étant enroulé en spirale autour du cathéter interne et étant lié de manière adhésive au cathéter interne avec un matériau adhésif, le matériau adhésif servant également à fournir une interaction collante entre les saillies et une surface interne du dispositif stent.
